# EUROPÄISCHE PATENTANMELDUNG

(11) **EP 3 756 705 A1**
(43) Veröffentlichungstag der Anmeldung: **30.12.2020**
(21) Anmeldenummer: 19183036.3
(22) Anmeldetag: 27.06.2019
(51) Int. Cl.: A61M 5/19, A61B 17/00

(54) **APPLIKATOR FÜR EINEN ZWEI-KOMPONENTEN GEWEBEKLEBER**

(71) Anmelder: Adhesys Medical GmbH, 52074 Aachen (DE)
(72) Erfinder: DORSCHEID, Ralf, 52134 Herzogenrath (DE); HAYES, Matthew, Boston, MA 02127 (US); LOUGH, Hayley A., Boston, MA 02199 (DE)
(74) Vertreter: Davepon, Björn

(57) **Zusammenfassung**

Die vorliegende Erfindung betrifft ein Dosiersystem (1) für ein wenigstens zwei Komponenten umfassendes Gewebekleber-System, umfassend einen in Längsrichtung in eine erste Kammer (2) und eine zweite Kammer (3) unterteilten Behälter, der im Bereich der am Behälterauslass (4) angeordneten ersten Kammer (2) ein biegesteif ausgestaltetes Schulterstück (5) aufweist, wobei die erste Kammer (2) einen Folienbeutel (6) zur Aufnahme der ersten Komponente des Gewebeklebersystems beinhaltet und der übrige Hohlraum des Behälterkörpers als zweite Kammer (3) zur Aufnahme der zweiten Komponente des Gewebeklebersystems ausgestaltet und durch einen flexiblen Behältermantel (7) umgeben ist, wobei sich ein Führungskanal (8) vom Behälterauslass (4) bis in die zweite Kammer (3) erstreckt, in dem ein Mischrohr (9) mit innenliegendem Statikmischer in Längsrichtung verschiebbar angeordnet ist und das Mischrohr (9) innerhalb des Führungskanals (8) in Längsrichtung wenigstens so weit aus dem Behälterauslass (4) herausgezogen werden kann, dass eine im Führungskanal (8) im Bereich der ersten Kammer (2) vorgesehene Austrittsöffnung (10) für die erste Komponente des Gewebeklebersystems zum Inkontaktbringen mit der zweiten Komponente des Gewebeklebersystems im Führungskanal (8) freigegeben wird. Die Erfindung betrifft ferner ein Kit aus einem Dosiersystem, welches mit einem wenigstens zwei Komponenten umfassendes Gewebekleber-System befüllt ist sowie ein Verfahren zum Verschließen einer Wunde mithilfe eines solchen Kits.

## Beschreibung

Die vorliegende Erfindung betrifft ein Dosiersystem mit wenigstens zwei Kammern für ein wenigstens zwei Komponenten umfassendes Gewebekleber-System. Die Erfindung betrifft ferner ein Kit aus einem Dosiersystem, welches mit einem wenigstens zwei Komponenten umfassendes Gewebekleber-System befüllt ist sowie ein Verfahren zum Verschließen einer Wunde mithilfe eines solchen Kits.

Aus dem Stand der Technik sind Zweikomponenten-Gewebekleber-Systeme (2K-Gewebekleber-Systeme) sowie entsprechende Dosiersysteme mit zwei Kammern bekannt. So offenbart die EP 2 173 782 B1 ein 2-Kammer-Dosiersystem welches mit einem 2K-Gewebekleber-System befüllt ist, von denen die eine Komponente ein isocyanatfunktionelles Präpolymer aus einem Polyisocyanat und einem Polyol und die zweite Komponente ein aminofunktioneller Asparaginsäureester ist. Solche 2-Kammer-Dosiersysteme werden üblicherweise als medizinische Spritze ausgestaltet mit zwei parallel zueinander angeordneten Zylindern, die synchron ausgepresst werden, wobei die zwei Komponenten am Ausgang der Spritze in einer Leitung zusammengeführt und über einen mit einem Statikmischer versehenen Applikator auf die gewünschte Stelle aufgetragen werden können.

Bei dieser Lösung kann es zum Teil als nachteilig empfunden werden, dass die Bedienung nur von Fachpersonal durchgeführt werden kann. Typischerweise werden hierfür auch beide Hände benötigt. Zudem sind solche spritzenartigen 2-Kammer-Dosiersysteme mit parallel zueinander angeordneten Zylindern mechanisch nicht sonderlich stabil und beispielsweise empfindlich gegen Verbiegen, was insbesondere durch die konstruktionsbedingte längliche und zugleich dünne Form begünstigt wird, da die Kolben der Spritze wegen der gefüllten Spritzenzylinder in der Regel voll ausgezogen sind.

Die Aufgabe der vorliegenden Erfindung bestand somit darin, ein Dosiersystem mit wenigstens zwei Kammern für ein wenigstens zwei Komponenten umfassendes Gewebekleber-System anzugeben, welches auch von nicht medizinisch vorgebildeten Personen bedient werden kann, vorzugsweise mit einer Hand, damit beispielsweise Verletzungen am eigenen Arm oder der eigenen Hand ebenfalls behandelt werden können. Vorzugsweise soll das Dosiersystem mechanisch stabiler als die bislang bekannten spritzenartigen 2-Kammer-Dosiersysteme aufgebaut sein.

Die Aufgabe wird gelöst durch ein Dosiersystem für ein wenigstens zwei Komponenten umfassendes Gewebekleber-System, umfassend einen in Längsrichtung in eine erste Kammer und eine zweite Kammer unterteilten Behälter, der im Bereich der am Behälterauslass angeordneten ersten Kammer ein biegesteif ausgestaltetes Schulterstück aufweist, wobei die erste Kammer einen Folienbeutel zur Aufnahme der ersten Komponente des Gewebeklebersystems beinhaltet und der übrige Hohlraum des Behälterkörpers als zweite Kammer zur Aufnahme der zweiten Komponente des Gewebeklebersystems ausgestaltet und durch einen flexiblen Behältermantel umgeben ist, wobei sich ein Führungskanal vom Behälterauslass bis in die zweite Kammer erstreckt, in dem ein Mischrohr mit innenliegendem Statikmischer in Längsrichtung verschiebbar angeordnet ist und das Mischrohr innerhalb des Führungskanals in Längsrichtung wenigstens so weit aus dem Behälterauslass herausgezogen werden kann, dass eine im Führungskanal im Bereich der ersten Kammer vorgesehene Austrittsöffnung für die erste Komponente des Gewebeklebersystems zum Inkontaktbringen mit der zweiten Komponente des Gewebeklebersystems im Führungskanal freigegeben wird.

Der Erfindung liegt die Erkenntnis zugrunde, dass durch Abtrennung der zwei Komponenten mittels einer Membran im Inneren der Dosiereinrichtung diese deutlich kompakter als die bislang bekannten spritzenartigen 2-Kammer-Dosiersysteme ausgelegt werden kann. Hierdurch kann das Dosiersystem beispielsweise eine flaschen- oder tubenförmige Geometrie besitzen die deutlich kürzer als eine befüllte 2-Kammer-Spritze vergleichbaren Inhalts ist. Dadurch wird es auch möglich, das Dosiersystem handergonomischer zu gestalten, sodass es auch von im Umgang hiermit un- oder wenig geübten Personen in einer Hand zusammengedrückt und das 2K-Gewebekleber-System auf diese Weise ausgepresst werden kann. Durch das biegesteif ausgestaltete Schulterstück wird zudem der innenliegende Folienbeutel, der üblicherweise mit dem Härter befüllt ist, vor unbeabsichtigtem Zusammendrücken und damit Platzen geschützt. Ebenso dient das Schulterstück dem Schutz der innenliegenden Mischeinrichtung.

Bevorzugt ist das erfindungsgemäße Dosiersystem mit zwei Kammern für ein zwei Komponenten umfassendes Gewebekleber-System ausgebildet.

Bei dem erfindungsgemäßen Dosiersystem kann das Mischrohr und/oder der Führungskanal mit einer Anschlageinrichtung zur Begrenzung des Verschiebeweges des Mischrohrs ausgerüstet sein. An der Anschlageinrichtung kann wiederum eine Rasteinrichtung vorgesehen sein, die ein unbeabsichtigtes Wiedereinschieben des Mischrohrs in den Führungskanal verhindert. Auf der außenseitigen Öffnung des Mischrohrs ist vorzugsweise ein Verschlussstopfen aufgesetzt, der insbesondere mit einer Rasteinrichtung versehen ist, deren Haltekraft besonders bevorzugt höher als die zum Herausziehen des Mischrohrs bis zur Anschlageinrichtung benötigte Kraft ist.

Bei dem erfindungsgemäßen Dosiersystem kann der Führungskanal in Längsrichtung mittig im Behälterkörper angeordnet und mit dem Behälterauslass fest verbunden sein. Das Schulterstück kann mit dem Behältermantel verschweißt, insbesondere mittels Reibschweißen, Ultraschallschallschweißen oder thermischem Schweißen, verpresst, verklebt, insbesondere mittels Heißkleben, oder vulkanisiert sein. Unabhängig hiervon kann der Folienbeutel mit Führungskanal auf der dem Behälterauslass abgewandten Seite der Austrittsöffnung fluiddicht verbunden sein, insbesondere verschweißt. Ferner unabhängig hiervon kann der Folienbeutel auf seiner dem Schulterstück zugewandten Seite im Bereich des Behälterauslasses mit diesem und/ oder dem Schulterstück fluiddicht verbunden sein, insbesondere verschweißt.

Hierdurch wird ein mechanisch fester Verbund gewährleistet, sodass auch bei der Verwendung des Dosiersystems keine Reaktanden an unbeabsichtigten Stellen austreten.

Das Material des Schulterstücks, des Folienbeutels und des Behältermantels kann unabhängig voneinander ausgewählt sein aus Kunststoffen, metallbeschichteten Kunststoffen, Metallfolien oder Verbundfolien aus den vorgenannten Materialien. Das Schulterstück ist insbesondere aus Polyethylen, Polypropylen, Polycarbonat, Polyester, Polyurethan, Polystyrol, Polyamid, Styrol-Acrylnitril oder einem Blend von diesen aufgebaut ist und unabhängig hiervon das Material des Folienbeutels und/ oder des Behältermantels aus einer aluminiumkaschierten Kunststofffolie ausgewählt.

Das erfindungsgemäße Dosiersystem besitzt einen Statikmischer. Statikmischer sind dem Fachmann grundsätzlich bekannt. Vorzugsweise besitzt der Statikmischer wenigstens 8 Mischelemente, vorzugsweise wenigstens 10 Mischelemente, besonders bevorzugt wenigstens 12 Mischelemente.

Das Volumenverhältnis der ersten Kammer zur zweiten Kammer richtet sich zweckmäßiger weise am stöchimetrischen Mischungsverhältnis der beiden Komponenten, also beispielsweise Härter und Präpolymer. Das Volumenverhältnis der ersten Kammer zur zweiten Kammer liegt typischerweise im Bereich von 1 : 2 bis 1 : 10, insbesondere von 1 : 2,5 bis 1 : 8, bevorzugt von 1 : 3 bis 1 : 6, ganz besonders bevorzugt bei etwa 1 : 4 ± 10%.

Ein weiterer Gegenstand der vorliegenden Erfindung betrifft ein Kit aus einem erfindungsgemäßen Dosiersystem und einem darin befindlichen zwei Komponenten umfassenden Gewebekleber-System, sowie optional eine Verfahrensanleitung zum Verschließen einer Wunde mithilfe des Kits, wobei das Gewebekleber-System bevorzugt wenigstens einen Asparaginsäureester als Härter mit optional wenigstens einem Füllstoff als erste Komponente und als zweite Komponente ein isocynatfunktionelles Präpolymer umfasst.

Die Erfindung betrifft ferner ein Verfahren zum Verschließen einer Wunde mithilfe eines erfindungsgemäßen Kits umfassend die Schritte:
a) Herausziehen und verriegeln (einrasten) des Mischrohrs bis zur Freigabe der Austrittsöffnung, insbesondere bis zum Erreichen der Anschlageinrichtung;
b) Druckausübung auf den Behältermantel, wodurch die zweite Komponente durch den Führungskanal gefördert, beim Passieren der Austrittsöffnung mit der ersten Komponente in Kontakt gebracht und beim Durchströmen des Mischrohres mit dieser vermischt wird;
c) Optionales Verwerfen der wenigstens ersten 10 mm aus dem Mischrohr austretenden Gewebeklebers;
d) Auftragen des unter weiterer Druckausübung aus dem Mischrohr austretenden Gewebeklebers auf die Wunde;
e) Aushärtenlassen des Gewebeklebers.

Die Erfindung betrifft insbesondere die folgenden Ausführungsformen:
Nach einer ersten Ausführungsform betrifft die Erfindung ein Dosiersystem 1 für ein wenigstens zwei Komponenten umfassendes Gewebekleber-System, umfassend einen in Längsrichtung in eine erste Kammer 2 und eine zweite Kammer 3 unterteilten Behälter, der im Bereich der am Behälterauslass 4 angeordneten ersten Kammer 2 ein biegesteif ausgestaltetes Schulterstück 5 aufweist, wobei die erste Kammer 2 einen Folienbeutel 6 zur Aufnahme der ersten Komponente des Gewebeklebersystems beinhaltet und der übrige Hohlraum des Behälterkörpers als zweite Kammer 3 zur Aufnahme der zweiten Komponente des Gewebeklebersystems ausgestaltet und durch einen flexiblen Behältermantel 7 umgeben ist, wobei sich ein Führungskanal 8 vom Behälterauslass 4 bis in die zweite Kammer 3 erstreckt, in dem ein Mischrohr 9 mit innenliegendem Statikmischer in Längsrichtung verschiebbar angeordnet ist und das Mischrohr 9 innerhalb des Führungskanals 8 in Längsrichtung wenigstens so weit aus dem Behälterauslass 4 herausgezogen werden kann, dass eine im Führungskanal 8 im Bereich der ersten Kammer 2 vorgesehene Austrittsöffnung 10 für die erste Komponente des Gewebeklebersystems zum Inkontaktbringen mit der zweiten Komponente des Gewebeklebersystems im Führungskanal 8 freigegeben wird.

Nach einer zweiten Ausführungsform betrifft die Erfindung ein Dosiersystem nach Ausführungsform 1, dadurch gekennzeichnet, dass das Mischrohr 9 und/ oder der Führungskanal 8 mit einer Anschlageinrichtung zur Begrenzung des Verschiebeweges des Mischrohrs 9 ausgerüstet ist. An der Anschlageinrichtung kann wiederum eine Rasteinrichtung vorgesehen sein, die ein unbeabsichtigtes Wiedereinschieben des Mischrohrs 9 in den Führungskanal 8 verhindert.

Nach einer dritten Ausführungsform betrifft die Erfindung ein Dosiersystem nach Ausführungsform 1 oder 2, dadurch gekennzeichnet, dass auf außenseitigen Öffnung des Mischrohrs 9 ein Verschlussstopfen 11 aufgesetzt ist, der vorzugsweise mit einer Rasteinrichtung versehen ist, deren Haltekraft besonders bevorzugt höher als die zum Herausziehen des Mischrohrs 9 bis zur Anschlageinrichtung benötigte Kraft ist.

Nach einer vierten Ausführungsform betrifft die Erfindung ein Dosiersystem nach einer der vorstehenden Ausführungsformen, dadurch gekennzeichnet, dass der Führungskanal 8 in Längsrichtung mittig im Behälterkörper angeordnet und mit dem Behälterauslass 4 fest verbunden ist.

Nach einer fünften Ausführungsform betrifft die Erfindung ein Dosiersystem nach einer der vorstehenden Ausführungsformen, dadurch gekennzeichnet, dass das Schulterstück 5 mit dem Behältermantel 7 verschweißt, insbesondere mittels Reibschweißen, Ultraschallschallschweißen oder thermischem Schweißen, verpresst, verklebt, insbesondere mittels Heißkleben, oder vulkanisiert ist.

Nach einer sechsten Ausführungsform betrifft die Erfindung ein Dosiersystem nach einer der vorstehenden Ausführungsformen, dadurch gekennzeichnet, dass der Behälter als Tube oder Flasche ausgestaltet ist.

Nach einer siebten Ausführungsform betrifft die Erfindung ein Dosiersystem nach einer der vorstehenden Ausführungsformen, dadurch gekennzeichnet, dass das Material des Schulterstücks 5, des Folienbeutels 6 und des Behältermantels 7 unabhängig voneinander ausgewählt ist aus Kunststoffen, metallbeschichteten Kunststoffen, Metallfolien oder Verbundfolien aus den vorgenannten Materialien, wobei das Schulterstück 5 insbesondere aus Polyethylen, Polypropylen, Polycarbonat, Polyester, Polyurethan, Polystyrol, Polyamid, Styrol-Acrylnitril oder einem Blend von diesen aufgebaut ist und unabhängig hiervon das Material des Folienbeutels 6 und/ oder des Behältermantels 7 aus einer aluminiumkaschierten Kunststofffolie ausgewählt ist.

Nach einer achten Ausführungsform betrifft die Erfindung ein Dosiersystem nach einer der vorstehenden Ausführungsformen, dadurch gekennzeichnet, dass der Statikmischer über wenigstens 8 Mischelemente verfügt, vorzugsweise über wenigstens 10 Mischelemente, besonders bevorzugt wenigstens 12 Mischelemente.

Nach einer neunten Ausführungsform betrifft die Erfindung ein Dosiersystem nach einer der vorstehenden Ausführungsformen, dadurch gekennzeichnet, dass der Folienbeutel 6 mit Führungskanal 8 auf der dem Behälterauslass 4 abgewandten Seite der Austrittsöffnung 10 fluiddicht verbunden ist, insbesondere verschweißt.

Nach einer zehnten Ausführungsform betrifft die Erfindung ein Dosiersystem nach einer der vorstehenden Ausführungsformen, dadurch gekennzeichnet, dass der Folienbeutel 6 auf seiner dem Schulterstück 5 zugewandten Seite im Bereich des Behälterauslasses 4 mit diesem und/ oder dem Schulterstück 5 fluiddicht verbunden ist, insbesondere verschweißt.

Nach einer elften Ausführungsform betrifft die Erfindung ein Dosiersystem nach einer der vorstehenden Ausführungsformen, dadurch gekennzeichnet, dass das Volumenverhältnis der ersten Kammer 2 zur zweiten Kammer 3 im Bereich von 1 : 2 bis 1 : 10 liegt, insbesondere von 1 : 2,5 bis 1 : 8, bevorzugt von 1 : 3 bis 1 : 6.

Nach einer zwölften Ausführungsform betrifft die Erfindung ein Kit aus einem Dosiersystem 1 nach einer der Ausführungsformen 1 bis 11, und einem darin befindlichen zwei Komponenten umfassenden Gewebekleber-System, sowie optional eine Verfahrensanleitung zum Verschließen einer Wunde mithilfe des Kits, wobei das Gewebekleber-System bevorzugt wenigstens einen Asparaginsäureester als Härter mit optional wenigstens einem Füllstoff als erste Komponente und als zweite Komponente ein isocynatfunktionelles Präpolymer umfasst.

Nach einer dreizehnten Ausführungsform betrifft die Erfindung ein Verfahren zum Verschließen einer Wunde mithilfe eines Kits gemäß Ausführungsform 12 umfassend die Schritte:
a) Herausziehen und verriegeln, insbesondere durch Einrasten, des Mischrohrs 9 bis zur Freigabe der Austrittsöffnung, insbesondere bis zum Erreichen der Anschlageinrichtung;
b) Druckausübung auf den Behältermantel 7, wodurch die zweite Komponente durch den Führungskanal 8 gefördert, beim Passieren der Austrittsöffnung 10 mit der ersten Komponente in Kontakt gebracht und beim Durchströmen des Mischrohres 9 mit dieser vermischt wird;
c) Optionales Verwerfen der wenigstens ersten 10 mm aus dem Mischrohr 9 austretenden Gewebeklebers;
d) Auftragen des unter weiterer Druckausübung aus dem Mischrohr 9 austretenden Gewebeklebers auf die Wunde;
e) Aushärtenlassen des Gewebeklebers.

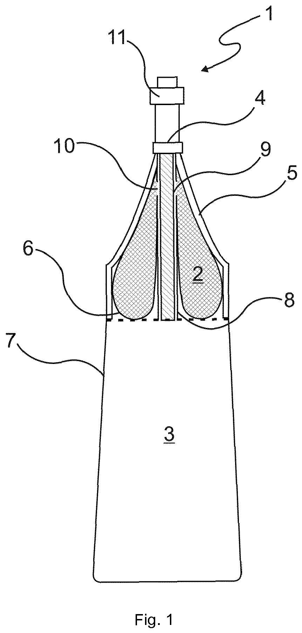

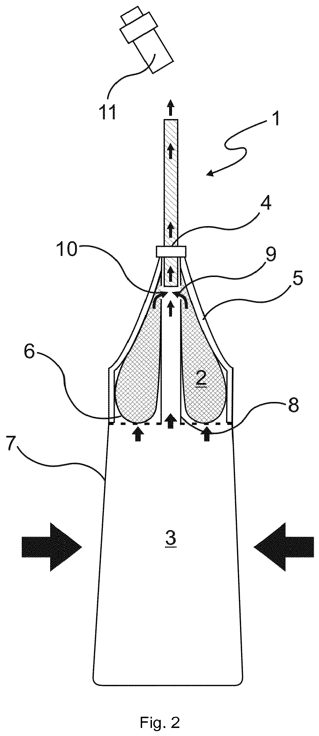

Die vorliegende Erfindung wird im Folgenden anhand eines in den Fign. 1 und 2 gezeigten Ausführungsbeispiels näher erläutert. Darin zeigt
- Fig. 1: eine Ausführungsform eines erfindungsgemäßen Dosiersystems in seitlicher Schnittdarstellung, sowie
- Fig. 2: das Dosiersystem aus Fig. 1 mit abgezogenem Verschlussstopfen und herausgezogenem Mischrohr.

In Fig. 1 ist ein Kit mit einem Dosiersystem 1 für ein wenigstens zwei Komponenten umfassendes Gewebekleber-System abgebildet. Das Dosiersystem 1 umfasst einen in Längsrichtung in eine erste Kammer 2 und eine zweite Kammer 3 unterteilten Behälter, der im Bereich der am Behälterauslass 4 angeordneten ersten Kammer 2 ein biegesteif ausgestaltetes Schulterstück 5 aufweist, wobei die erste Kammer 2 einen Folienbeutel 6 zur Aufnahme der ersten Komponente des Gewebeklebersystems umfasst und der übrige Hohlraum des Behälterkörpers als zweite Kammer 3 zur Aufnahme der zweiten Komponente des Gewebeklebersystems ausgestaltet und von einem flexiblen Behältermantel 7 umgeben ist. Das Gewebekleber-System besteht aus einem Asparaginsäureester als Härter mit optional wenigstens einem Füllstoff als erste Komponente und einem isocynatfunktionellen Präpolymer als zweite Komponente. Das Volumenverhältnis der ersten Kammer 2 zur zweiten Kammer 3 beträgt vorliegend etwa 1 : 4, was dem optimalen Mischungsverhältnis des Asparaginsäureesters als Härter und dem isocynatfunktionellen Präpolymer entspricht.

Das Material des Behältermantels 7 besteht aus einer aluminiumkaschierten Kunststofffolie, um den Inhalt vor dem Zutritt von Feuchtigkeit und Sauerstoff zu schützen. Hierdurch ist eine gesonderte gasdichte Umverpackung nicht zwingend notwendig. Das biegesteife Schulterstück 5 ist mit dem Behältermantel 7 verschweißt, besteht aus Polypropylen und erstreckt sich in Längsrichtung vom Behälterauslass 4 bis zur maximalen Längsausdehnung des Folienbeutels 6, was vorliegend mit einer gedachten gestrichelten Linie dargestellt ist. Auf diese Weise ist der Folienbeutel 6 durch das Schulterstück 5 gegen unbeabsichtigtes Zusammendrücken geschützt. Ferner ist der Folienbeutel 6 auf seiner dem Schulterstück 5 zugewandten Seite im Bereich des Behälterauslasses 4 mit diesem und dem Schulterstück 5 fluiddicht verschweißt. Hierdurch wird der Inhalt des Folienbeutels 6 vor Feuchtigkeits- und Sauerstoffzutritt geschützt und zudem ein unbeabsichtigtes Vermischen der beiden Komponenten aus den beiden Kammer 2, 3 verhindert.

Im Inneren des Behälter erstreckt sich ein mittig im Behälterkörper angeordneter Führungskanal 8 vom Behälterauslass 4 bis in die zweite Kammer 3, ebenfalls bis zu der gedachten gestrichelten Linie, wobei der Führungskanal 8 mit dem Behälterauslass 4 fest verbunden ist. In dem Führungskanal 8 ist ein Mischrohr 9 mit innenliegendem Statikmischer mit 12 feststehenden Mischelementen in Längsrichtung verschiebbar angeordnet. Die Länge des Mischrohrs 9 beträgt etwa 8 cm. Der Folienbeutel 6 ist mit dem Führungskanal 8 auf der dem Behälterauslass 4 abgewandten Seite der Auslassöffnung fluiddicht verschweißt. Das Mischrohr 9 kann innerhalb des Führungskanals 8 in Längsrichtung so weit aus dem Behälterauslass 4 herausgezogen werden, dass eine im Führungskanal 8 im Bereich der ersten Kammer 2 vorgesehene Austrittsöffnung 10 für die erste Komponente des Gewebeklebersystems zum Inkontaktbringen mit der zweiten Komponente des Gewebeklebersystems im Führungskanal 8 freigegeben wird. Die Begrenzung des Auszugweges wird durch eine an dem Mischrohr 9 und dem Führungskanal 8 vorgesehene Anschlageinrichtung realisiert (vorliegend nicht dargestellt). An der Anschlageinrichtung kann wiederum eine Rasteinrichtung vorgesehen sein, die ein unbeabsichtigtes Wiedereinschieben des Mischrohrs 9 in den Führungskanal 8 verhindert.

Auf der außenseitigen Öffnung des Mischrohrs 9 ist ein Verschlussstopfen 11 aufgesetzt, der mit einer Rasteinrichtung versehen ist, deren Haltekraft höher als die zum Herausziehen des Mischrohrs 9 bis zur Anschlageinrichtung benötigte Kraft ist. Um ein unbeabsichtigtes Herausziehen des Mischrohrs 9 zu verhindern, ist dieses und/ oder der Führungskanal 8 mit einer Rasteinrichtung ausgerüstet, wobei deren Haltekraft wiederum niedriger ist als diejenige der Rasteinrichtung des Verschlussstopfens 11, damit beim Ziehen zunächst das Mischrohr 9 herausgezogen wird bevor der Verschlussstopfen 11 abgezogen werden kann.

In Fig. 2 ist die Verwendung des Kits beziehungsweise ein Verfahren zum Verschließen einer Wunde mit dem Kit aus Fig. 1 dargestellt. Hierzu wird das Dosiersystem 1 in der einen Hand gehalten und mit der anderen am Verschlussstopfen 11 gezogen. Hierdurch wird in einem ersten Schritt das Mischrohr 9 so weit aus dem Führungskanal 8 herausgezogen, bis die Anschlageinrichtung ein weiteres Herausziehen verhindert. Durch weiteres Ziehen wird die Haltekraft der Rasteinrichtung des Verschlusstopfens 11 überwunden und diese so abgezogen. Die Handhabung kann auch einhändig erfolgen, beispielsweise wenn eine Hand oder ein Arm verletzt ist. In diesem Fall wird der Verschlussstopfen 11 beispielsweise mit den Zähnen gezogen.

Durch das Herausziehen des Mischrohrs 9 wird die Austrittsöffnung 10 freigegeben, sodass bei der Ausübung von Druck mit der das Dosiersystem 1 haltenden Hand das in der zweiten Kammer 3 befindliche isocyanatfunktionelle Präpolymer in den Führungskanal 8 und das Mischrohr 9 befördert wird. Gleichzeitig wird hierdurch Druck auf den die erste Kammer 2 bildenden Folienbeutel 6 ausgeübt, wodurch der darin befindliche Asparaginsäureester über die Austrittsöffnung 10 ebenfalls in das Mischrohr 9 befördert wird. Bei weiterer Druckausübung werden die Komponenten durch das Mischrohr 9 in Richtung der Austrittsöffnung 4 befördert und dabei durch die Mischelemente weitestgehend homogen vermischt, sodass sie nach dem Verlassen der Austrittsöffnung direkt auf die zu verschließende Wunde als Reaktionsmischung aufgebracht werden können, um dort auszuhärten. Die Verarbeitungszeit (die sog. "pot life") beträgt beispielsweise 180 bis 360 s und die Zeit bis zur Aushärtung etwa 5 min, wobei beides durch entsprechende Anpassungen an den Reaktanden schneller oder langsamer eingestellt werden kann.

### Bezugszeichenliste:

- 1: Dosiersystem
- 2: Kammer
- 3: Zweite Kammer
- 4: Behälterauslass
- 5: Schulterstück
- 6: Folienbeutel
- 7: Behältermantel
- 8: Führungskanal
- 9: Mischrohr
- 10: Austrittsöffnung
- 11: Verschlussstopfen

## Patentansprüche

1. Dosiersystem (1) für ein wenigstens zwei Komponenten umfassendes Gewebekleber-System, umfassend einen in Längsrichtung in eine erste Kammer (2) und eine zweite Kammer (3) unterteilten Behälter, der im Bereich der am Behälterauslass (4) angeordneten ersten Kammer (2) ein biegesteif ausgestaltetes Schulterstück (5) aufweist, wobei die erste Kammer (2) einen Folienbeutel (6) zur Aufnahme der ersten Komponente des Gewebeklebersystems beinhaltet und der übrige Hohlraum des Behälterkörpers als zweite Kammer (3) zur Aufnahme der zweiten Komponente des Gewebeklebersystems ausgestaltet und durch einen flexiblen Behältermantel (7) umgeben ist, wobei sich ein Führungskanal (8) vom Behälterauslass (4) bis in die zweite Kammer (3) erstreckt, in dem ein Mischrohr (9) mit innenliegendem Statikmischer in Längsrichtung verschiebbar angeordnet ist und das Mischrohr (9) innerhalb des Führungskanals (8) in Längsrichtung wenigstens so weit aus dem Behälterauslass (4) herausgezogen werden kann, dass eine im Führungskanal (8) im Bereich der ersten Kammer (2) vorgesehene Austrittsöffnung (10) für die erste Komponente des Gewebeklebersystems zum Inkontaktbringen mit der zweiten Komponente des Gewebeklebersystems im Führungskanal (8) freigegeben wird.

2. Dosiersystem nach Anspruch 1, **dadurch gekennzeichnet, dass** das Mischrohr (9) und/ oder der Führungskanal (8) mit einer Anschlageinrichtung zur Begrenzung des Verschiebeweges des Mischrohrs (9) ausgerüstet ist.

3. Dosiersystem nach Anspruch 1 oder 2, **dadurch gekennzeichnet, dass** auf außenseitigen Öffnung des Mischrohrs (9) ein Verschlussstopfen (11) aufgesetzt ist, der vorzugsweise mit einer Rasteinrichtung versehen ist, deren Haltekraft besonders bevorzugt höher als die zum Herausziehen des Mischrohrs (9) bis zur Anschlageinrichtung benötigte Kraft ist.

4. Dosiersystem nach einem der vorstehenden Ansprüche, **dadurch gekennzeichnet, dass** der Führungskanal (8) in Längsrichtung mittig im Behälterkörper angeordnet und mit dem Behälterauslass (4) fest verbunden ist.

5. Dosiersystem nach einem der vorstehenden Ansprüche, **dadurch gekennzeichnet, dass** das Schulterstück (5) mit dem Behältermantel (7) verschweißt, insbesondere mittels Reibschweißen, Ultraschallschallschweißen oder thermischem Schweißen, verpresst, verklebt, insbesondere mittels Heißkleben, oder vulkanisiert ist.

6. Dosiersystem nach einem der vorstehenden Ansprüche, **dadurch gekennzeichnet, dass** der Behälter als Tube oder Flasche ausgestaltet ist.

7. Dosiersystem nach einem der vorstehenden Ansprüche, **dadurch gekennzeichnet, dass** das Material des Schulterstücks (5), des Folienbeutels (6) und des Behältermantels (7) unabhängig voneinander ausgewählt ist aus Kunststoffen, metallbeschichteten Kunststoffen, Metallfolien oder Verbundfolien aus den vorgenannten Materialien, wobei das Schulterstück (5) insbesondere aus Polyethylen, Polypropylen, Polycarbonat, Polyester, Polyurethan, Polystyrol, Polyamid, Styrol-Acrylnitril oder einem Blend von diesen aufgebaut ist und unabhängig hiervon das Material des Folienbeutels (6) und/ oder des Behältermantels (7) aus einer aluminiumkaschierten Kunststofffolie ausgewählt ist.

8. Dosiersystem nach einem der vorstehenden Ansprüche, **dadurch gekennzeichnet, dass** der Statikmischer über wenigstens 8 Mischelemente verfügt, vorzugsweise über wenigstens 10 Mischelemente, besonders bevorzugt wenigstens 12 Mischelemente.

9. Dosiersystem nach einem der vorstehenden Ansprüche, **dadurch gekennzeichnet, dass** der Folienbeutel (6) mit Führungskanal (8) auf der dem Behälterauslass (4) abgewandten Seite der Austrittsöffnung (10) fluiddicht verbunden ist, insbesondere verschweißt.

10. Dosiersystem nach einem der vorstehenden Ansprüche, **dadurch gekennzeichnet, dass** der Folienbeutel (6) auf seiner dem Schulterstück (5) zugewandten Seite im Bereich des Behälterauslasses (4) mit diesem und/ oder dem Schulterstück (5) fluiddicht verbunden ist, insbesondere verschweißt.

11. Dosiersystem nach einem der vorstehenden Ansprüche, **dadurch gekennzeichnet, dass** das Volumenverhältnis der ersten Kammer (2) zur zweiten Kammer (3) im Bereich von 1 : 2 bis 1 : 10 liegt, insbesondere von 1 : 2,5 bis 1 : 8, bevorzugt von 1 : 3 bis 1 : 6.

12. Kit aus einem Dosiersystem (1) nach einem der Ansprüche 1 bis 11, und einem darin befindlichen zwei Komponenten umfassenden Gewebekleber-System, sowie optional eine Verfahrensanleitung zum Verschließen einer Wunde mithilfe des Kits, wobei das Gewebekleber-System bevorzugt wenigstens einen Asparaginsäureester als Härter mit optional wenigstens einem Füllstoff als erste Komponente und als zweite Komponente ein isocynatfunktionelles Präpolymer umfasst.

13. Verfahren zum Verschließen einer Wunde mithilfe eines Kits gemäß Anspruch 12 umfassend die Schritte:
a) Herausziehen und verriegeln des Mischrohrs (9) bis zur Freigabe der Austrittsöffnung, insbesondere bis zum Erreichen der Anschlageinrichtung;
b) Druckausübung auf den Behältermantel (7), wodurch die zweite Komponente durch den Führungskanal (8) gefördert, beim Passieren der Austrittsöffnung (10) mit der ersten Komponente in Kontakt gebracht und beim Durchströmen des Mischrohres (9) mit dieser vermischt wird;
c) Optionales Verwerfen der wenigstens ersten 10 mm aus dem Mischrohr (9) austretenden Gewebeklebers;
d) Auftragen des unter weiterer Druckausübung aus dem Mischrohr (9) austretenden Gewebeklebers auf die Wunde;
e) Aushärtenlassen des Gewebeklebers.
